# EUROPEAN PATENT SPECIFICATION

(11) **EP 0 437 556 B1**
(45) Date of publication and mention of the grant of the patent: **11.12.1996**
(21) Application number: 90909538.2
(22) Date of filing: 28.06.1990
(51) Int. Cl.: C07K 14/46, A61K 38/17

(54) **SUBSTITUTION ANALOGUES OF MAGAININ PEPTIDES**
SUBSTITUIERUNGSANALOGE VON MAGAININPEPTIDEN
ANALOGUES DE SUBSTITUTION DES PEPTIDES DE LA MAGAININE

(30) Priority: 07.07.1989 US 376754
(43) Date of publication of application: 24.07.1991
(60) Divisional application: 96201383.5
(73) Proprietor: THE SCRIPPS RESEARCH INSTITUTE, La Jolla, CA 92037 (US)
(72) Inventor: HOUGHTEN, Richard, A., Solana Beach, CA 92075 (US); CUERVO, Julio, H., La Jolla, CA 92037 (US)
(74) Representative: Fisher, Adrian John
(86) International application number: US9003675
(87) International publication number: WO9100869

(56) References cited:
- US-A- 4 810 777
- FEBS LETTERS, vol. 236, no. 2, August 1988, pages 462-466, published by Elsevier Science Publishers B.V. (Biomedical Division), Federation of European Biochemical Societies; H.-C. CHEN et al.: "Synthetic magainin analogues with improved antimicrobial activity"
- PEPTIDE RESEARCH, vol. 1, no. 2, November/December 1988, pages 81-86; J.H. CUERVO et al.: "The magainins: sequence factors relevant to increased antimicrobial activity and decreased hemolytic activity"
- PROCEEDINGS OF THE ELEVENTH AMERICAN PEPTIDE SYMPOSIUM, 9th-14th July 1989, La Jolla, California, Peptides - Chemistry, Structure and Biology, edited by J. E. Rivier et al., 1990, pages 124-126, ESCOM, Leiden, NL; J.H. CUERVO et al.: "Synthesis and antimicrobial activity of magainin alanine substitution analogs"
- PROCEEDINGS OF THE ELEVENTH AMERICAN PEPTIDE SYMPOSIUM, 9th-14th July 1989, La Jolla, California, Peptides - Chemistry, Structure and Biology, edited by J. E. Rivier et al., 1990, pages 124-126, ESCOM, Leiden, NL; H.-C. CHEN et al.: "Magainin analogs: A study of activity as a function of alpha-helix modification"
- DERWENT (WPIL), AN - 90-019331 [03]; & JP-A-88 127 177 (SAGAMI CHEM. RES. CENTRE) 26-05-1988
- INT. J. PEPTIDES PROTEIN RES., vol. 35, no. 2, February 1990, pages 141-146; F.S. TJOENG et al.: "Multiple peptide synthesis using a single support (MPS3)"
- Proc. Natl. Acad. Sci., "Antimicrobial Activity of Synthetic Magainin Peptides and Several Analogues", Volume 85, pp. 910-913, (ZASLOFF et al) Feb. (1988) see entire document.

## Description

This invention relates to a class of biologically active peptides known as magainins. More particularly, this invention relates to analogues of magainin peptides wherein at least one amino acid residue in the peptide has been substituted with another amino acid residue, with said analogues being commonly referred to as "substitution analogues".

H-C Chen *et al,* FEBS Letters, 236 (2), 462-466 (1988) disclose a limited range of substitution analogues of Magainin II (mostly involving alanine substitutions at residues 8, 13 and 18) , with improved antimicrobial activity. J.H. Cuervo *et al,* Peptide Research, 1 (2), 81-86 (1988) disclose amino acid omission analogues of Magainin I and Magainin II.

In accordance with the present invention, there is provided a compound comprising an analogue of Magainin I peptide or Magainin II peptide. The Magainin I or Magainin II peptide is in an amide or carboxy terminated form. Magainin I is represented by the following structural formula using the single letter amino acid code and the numbers below each amino acid residues refer to the position of the residue in the peptide. 
G I G K F L H S A G K F G K A F V G E I M K S
1 2 3 4 5 6 7 8 9 10 11 12 13 14 15 16 17 18 19 20 21 22 23
 Magainin II is represented by the following structural formula using the single letter amino acid code and the numbers below each amino acid residue refer to the position of the residue in the peptide:

G I G K F L H S A K K F G K A F V G E I M N S
1 2 3 4 5 6 7 8 9 10 11 12 13 14 15 16 17 18 19 20 21 22 23

In the analogues of the present invention, any of amino acid residues 15-23 of Magainin I peptide or Magainin II peptide is optionally omitted, and one to seven of the remaining amino acid residues of said Magainin peptide is substituted by another amino acid residue, at least one of amino acid residues 3, 7, 8, 10, 18-21 and 23 (in the case of Magainin I) or at least one of amino acid residues 1-3, 5-8, 12, 13 and 15-23 (in the case of Magainin II) being substituted by lysine and any further substitutions being as shown in the following table, provided that residue 18 may be substituted by Phe only if one of residues 15 to 17 or 19 to 23 is omitted.

| Residue No. | Substituent |
|---|---|
| 1 | Lys, Ala |
| 2 | Lys, Ala, D-Ile, Arg, Leu, Val, His, Met |
| 3 | Lys, Ala, Trp, Arg, His |
| 4 | D-Lys, Ala, Arg, His |
| 5 | D-Phe, Ala, Lys, Trp, Leu, Ile, Val, Met |
| 6 | D-Leu, Lys, Ala, Ile, Val, Met |
| 7 | Lys, D-His, Ala, Arg, Ile, Val, Met |
| 8 | Ala, Lys, D-Ser, Trp, Met, Ile, Arg, His, Thr, Leu, Val |
| 9 | Lys, D-Ala, Trp, Arg, His, Leu, Ile, Val |
| 10 | D-Lys, Lys, Ala, Trp, Arg, His, Leu, Ile, Val |
| 11 | D-Lys, Arg, His |
| 12 | D-Phe, Lys, Trp, Arg, His |
| 13 | Ala, Lys, Trp, Met, Arg, His, Phe, Leu, Ile, Val |
| 14 | Ala, D-Lys, Arg, His |
| 15 | Lys, Trp, D-Ala, Arg, His, Phe |
| 16 | Ala, Lys, D-Phe, Ile, Val, Met, Leu |
| 17 | Ala, Lys, D-Val, Trp, Arg, His, Met, Leu |
| 18 | Ala, Lys, Trp, Arg, His, Leu, Met, Phe |
| 19 | Ala, Lys, D-Glu, Gly, Arg, His, Leu Ile, Phe, Asn |
| 20 | D-Ile, Ala, Lys |
| 21 | Lys, Pro, Ala, His, Leu, Arg, Ile, Phe |
| 22 | Lys, Ala, D-Asn, Gln, Arg, His |
| 23 | D-Ser, Lys, Ala, Thr, Gly, Leu, Ile, Gln, Asn |

It is to be understood that for purposes of the present invention, the tryptophan residues may be protected with a formyl group or be unprotected. The phenylalanine residues, whether such residue is present in its normal position, or employed as a substitution residue, may be a normal phenylalanine residue or an iodinated phenylalanine residue.

In accordance with one embodiment, at least one of amino acid residues 3, 7, 8, 10, 18-21, and 23 of Magainin I is substituted with a lysine residue.

In accordance with another embodiment, amino acid residues 3, 8, 9, 19 and 23 of Magainin I are each substituted with a lysine residue and amino acid residue 16 is substituted with an alanine residue.

In accordance with another embodiment, at least one of amino acid residues 1-3, 5-8, 12, 13 and 15-23 of Magainin II is a lysine residue.

In accordance with yet another embodiment, amino acid residue 19 of Magainin I is deleted, and amino acid residues 5, 8, 9 and 16 are each substituted with a lysine residue, amino acid residue 21 is substituted with a leucine residue, and amino acid residues 18 and 23 are substituted with an alanine residue.

In accordance with another embodiment, in carboxy- or amide-terminated (preferably amide-terminated) Magainin I or Magainin II peptide, amino acid residues 17-23 or 16-23 or 15-23 are deleted, and amino acid residues 3, 7, and 8 are each substituted with a lysine residue, and optionally amino acid residue 13 and/or amino acid residue 10 is substituted with an alanine residue. Preferred peptides are as follows: GIKKFLKKAGKFGK-NH2 GIKKFLKKAGKFGKAF-NH2 GIKKFLKKAKKFGKA-NH2 GIKKFLKKAKKFAKA-NH2 GIKKFLKKAAKFAKA-NH2 Preliminary studies indicate that the above-mentioned preferred peptides possess low hemolytic activity. In accordance with a further embodiment, amino acid residue 21 of Magainin I may be deleted, and amino acid residues 5,10, 18, and 19 are each substituted with a lysine residue, amino acid residue 7 is substituted with a phenylalanine residue, and amino acid residue 22 is substituted with an alanine residue.

In accordance with another embodiment, in carboxy or amide terminated (preferably amide terminated) Magainin I or Magainin II (preferably Magainin II)analogs of the invention as defined before, amino acid residue 19 is omitted, and at least one of amino acid residues 3, 7, 8, 10, 13, 15, 16, 18 21, 22 or 23 is substituted with another amino acid as follows:

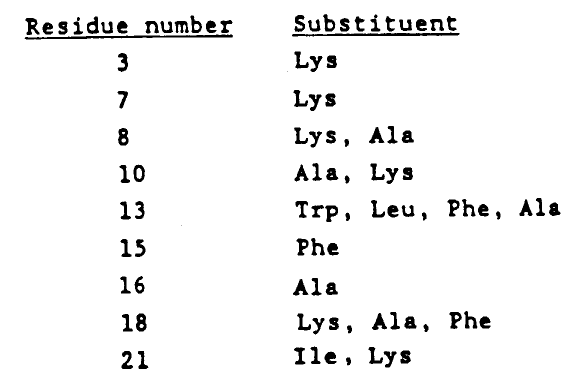

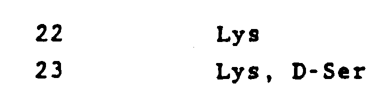

Representative peptides are disclosed in Examples 14 and 21-23.

In a preferred embodiment, the peptide is a Magainin II peptide, and the substitution analogue wherein amino acid 19 is deleted is selected from the class consisting of the following substitution analogues: GIGKFLHSAKKFGKAFVGIMKS; GIGKFLHSAKKFGKAFVAIMKS; GIGKFLKSAKKFGKAFVFIMNS; GIGKFLHKAKKFAKAFVFIMNS; GIGKFLKSAKKFAKAFVFIMNS; GIGKFLHKAKKFAKAFVFIMNK; GIGKFLKKAKKFGKAFVFIMKK; and GIGKFLHSAKKFGKAFVK**IMNS; wherein K** is E-F-moc-lysine.
Preliminary studies indicate that these preferred analogues possess low hemolytic activity.

Applicants have found that when employing the substitution analogues of Magainin I or Magainin II as hereinabove described, such peptides display biological activity equal to or greater than the parent Magainin I or Magainin II peptide. Such peptides are referred to as "successful substitution analogues".

The use of these compounds which comprise a substitution analogue of Magainin I or Magainin II peptide, in accordance with the present invention, is effective as an antibiotic, and can be employed to inhibit, prevent or destroy the growth or proliferation of microbes, such as bacteria, fungi viruses or the like. Similarly, such compounds can be employed as an anti-viral composition to inhibit, prevent or destroy the growth or proliferation of viruses.

As used herein, the term "substitution analogue" includes magainin peptides in which at least one amino acid residue of the peptide structure has been substituted with a different amino acid residue, as well as magainin peptides in which, in addition to the above-mentioned substitution(s), also have had at least one of amino acid residues 15-23 omitted from the peptide sequence. The term also includes magainin peptides having D-amino acid or iodinated phenylalanine residues which have been substituted into the peptide sequence.

Such compounds can also be employed as a spermicide to inhibit, prevent or destroy the motility of sperm.

Such compounds can also be employed as anti-tumor agents to inhibit the growth of or destroy tumors.

The compounds have a broad range of potent antibiotic activity against a plurality of microorganisms, including Gram-positive and Gram-negative bacteria, fungi, protozoa and the like. Such compounds can be employed for treating or controlling microbial infection caused by organisms which are sensitive to such compounds.

The compounds can also be used as preservatives or sterilants for materials susceptible to microbial contamination. In vitro activity against bacteria is exemplified hereinafter in Examples 3-27.

In general, a substitution analogue of the Magainin I or Magainin II peptide is administered in a dosage of from about 1 mg to about 500 mg per kilogram of body weight, when administered systemically. When administered topically, the peptide is used in a concentration of from about 0.5% to about 5%.

The compounds comprising the substitution analogues of Magainin I or Magainin II, in accordance with the present invention, can be employed for treating a wide variety of hosts. In accordance with a preferred embodiment, a host can be an animal, and such animal can be a human or non-human animal.

The compounds comprising the substitution analogues of Magainin I or Magainin II can be employed in a wide variety of pharmaceutical compositions in combination with a non-toxic pharmaceutical carrier or vehicle such as a filler, non-toxic buffer, or physiological saline solution. Such pharmaceutical compositions can be used topically or systemically and can be in any suitable form such as a liquid, solid, semi-solid, injectable solutions, tablet, ointment, lotion, paste, capsule or the like. The compounds comprising the substitution analogues of Magainin I or Magainin II can also be used in combination with adjuvants, protease inhibitors, or compatible drugs where such a combination is seen to be desirable or advantageous in controlling infection caused by harmful microorganisms including protozoa, viruses, and the like.

The compounds comprising the substitution analogues of Magainin I or Magainin II of the present invention can be administered to a host; in particular an animal, in an effective antibiotic and/or anti-tumor and/or anti-viral and/or anti-microbial and/or a spermicidal amount.

Magainin I and Magainin II, as well as the substitution analogues of the Magainin I and Magainin II peptides of the present invention, (both amide-and carboxy-terminated Magainin I and Magainin II forms) can be synthesized by any convenient method of peptide synthesis as are well-known to skilled workers. Solid phase synthesis methods are particularly preferred.

The peptides described herein were prepared by the method of simultaneous multiple peptide synthesis (SMPS). This method is described in detail in Houghten, R.A., "General Method for the Rapid Solid-Phase Synthesis of Large Numbers of Peptides; Specificity of Antigen-Antibody Interaction at the Level of Individual Amino Acids," Proc. Natl. Acad. Sci., U.S.A., Vol. 82, pgs. 5131-5135 (1985), and in Houghten, R.A., et al. "Simultaneous Multiple Peptide Synthesis; The Rapid Preparation of Large Numbers of Discrete Peptides for Biological, Immunological, and Methodological Studies," Peptide Chemistry, pgs. 295-298 (1987).

For purposes of the following examples, substitution analogues of Magainin I and Magainin II were prepared wherein various amino acid residues were substituted and/or other amino acid residue(s) were deleted.

For purposes of comparison, a complete Magainin I or Magainin II peptide can also be prepared by the SMPS method. It is also contemplated within the scope of the present invention that substitution analogues which have one or more amino acid residue(s) omitted from the Magainin I or Magainin II structure can also be prepared by the SMPS method.

The invention will now be described with respect to the following examples, however, the scope of the present invention is not intended to be limited thereby.

### Example 1 - Peptide Synthesis

Peptide synthesis of Magainin I and the substitution analogues of Magainin I, was accomplished by using the strategy of simultaneous multiple peptide synthesis. All solvents and reagents were of analytical grade and were used without further purification. Standard N-t-Boc-protected amino acids were employed in the synthesis. The side chain functionalities used were benzyl (Ser, Glu), 2-C1-Z (Lys)(Z=benzyloxycarbonyl), N^{im}- DNP (His), and sulfoxide (Met). Peptide synthesis was performed beginning with 100 mg of either Boc-amino acids-Pam resin to produce C-terminal carboxyl peptide (PAM purchased from Applied Biosystems, substitution 0.56 meq/gm is an amino acyl -4- [oxymethyl] phenylacetic acid derivative of amino polystyrene) or methylbenzhydrylamine (MBHA) resin (substitution-0.65meq/gm) per resin packet to produce C-terminal amide peptide.

After synthesis, completely protected peptide resins were treated three times with 0.5 M thiophenol in DMF to remove the N^{im}- dinitrophenyl group from Histidine. The final Boc-group was removed with TFA to avoid t-butylation of methionyl residues during final HF treatment. Cleavage was performed using the Low-High HF procedure. Tam, et al. J. Am. Chem. Soc. Vol. 105, P. 6442 (1983). For peptides synthesized on Pam resin, the low-HF was carried out without removing the resin from the packet, using a multiple vessel HF apparatus for 2 hrs. at 0°C. For peptides prepared using MBHA resin, the low HF procedure was performed in a common reaction vessel for 2 hrs. at 0°C. For Pam resin peptides, the low-HF mixture was evacuated from the 24 individual reaction vessels by a water aspirator followed by a mechanical pump. The low-HF reaction vessel containing the bags with MBHA resin was emptied of the low-HF mixture by pouring off the liquid into a waste container. The bags were washed immediately with cold ether followed by alternating washes of CH₂Cl₂, DMF, CH₂Cl₂, IPA, CH₂Cl₂. The packets were then dried and put into individual tubes of the 24 vessel HF apparatus with 0.7 ml of anisole as scavenger. The high-HF was performed by condensing dry hydrogen fluoride at -70°C. The reaction took place at -10°C for 1 h. and -5°C - 0°C for the last 30 min. HF was evaporated using a strong flow of nitrogen. Finally, residual carbonium ion scavengers were removed by washing with dry ether.

The crude peptides were subsequently extracted with 10% acetic acid and subjected to RP-HPLC on an analytical reversed phase column (Vidac ODS 25 cm x 4.6 mm), using a Beckman-Altek model 421 HPLC system and two model 110A pumps. The solvent system was composed of buffer A, 0.05% TFA/H₂O, and buffer B, 0.05% TFA/CH₃CN with a flow rate of 1.0 ml/min. The peptides were detected at 215 nm using a Hitachi 100-20 spectrophotometer.

Purification of the peptides was accomplished by reverse - phase HPLC on a Vidac C₁₈ (22 mm x 25cm), 10 µm packing column with an eluting gradient composed of CH₃CN and 0.05% TFA. Amino acid analysis was carried out on a Beckman 6300 analyzer following hydrolysis of the peptides in constant (boiling) 6 N HCl at 110°C for 24 hr., and such analysis was within =10% of theory.

### Example 2 - Antimicrobial Assays

Antimicrobial assays were carried out in 96 - well tissue culture plates. Each well was incubated with a given microorganism (Escherichia coli, Staphylococcus epidermidis, Staphylococcus aureus, or Pseudomonas aeruginosa) suspended in LB medium (Examples 3-17), or TSB medium (Examples 18-27). Upon the addition of Magainin I or its substitution analogues, (dissolved in 1 X PBS, pH 7.0) each well contained a final cell density of 1.0 x 10⁶ colony forming units (CFU)/ml. The final peptide concentrations used were 100 µg/ml., 75.0 µg/ml, 50.0 µg/ml, 25 µg/ml, 12.5 µg/ml, 10 µg/ml, 5µg/ml, 2.5 µg/ml, and 1.25 µg/ml.

Addition of peptides to the wells was defined as time zero. At six hours, the plates were placed in a Titertek Multiskan apparatus and the O.D.₆₂₀ determined. The plates as well as the initial innoculum were incubated at 37°C.

Five wells per plate contained media alone, while five others contained medium plus cells. These controls were used to eliminate the possibility of media contamination while providing a measure of uninhibited growth of the microorganisms.

The degree of peptide activity was determined by comparing the substitution analogues with uninhibited growth of the control cells over a six-hour period. The effective growth inhibition of the substitution analogues is listed in the examples and tables below.

### Example 3 - Magainin I analogues with alanine substitutions (comparative)

Magainin I and analogues wherein an alanine residue was substituted for various amino acid residues of Magainin I were prepared as hereinabove described in Example 1 and tested for % effective growth inhibition of E. coli at a concentration given in µg/ml as hereianbove described in Example 2. The % effective growth inhibition at a concentration in µg/ml for the alanine-substituted Magainin I analogues is listed below in Table I. As used herein, the heading "Amino Acid Residue Substituted" refers to the number of the amino acid residue in the peptide which is substituted with a desired amino acid residue. All other residues in the peptide remain the same as that of the normal peptide sequence. For purposes of explanation of the term "% effective growth inhibition," a value of "100 at 25", for example, indicates 100% effective growth inhibition at a concentration of 25 µg/ml.

**Table 1 -**

| Alanine-Substituted Magainin I Analogues | |
|---|---|
| Amino Acid Residue Substituted | % Effective Growth Inhibition Concentration in µg/ml |
| None (Magainin I) | 100 at 25 |
| 23 | 100 at 10 |
| 22 | 100 at 50 |
| 21 | 100 at 50 |
| 20 | 100 at 50 |
| 19 | 100 at 5 |
| 18 | 100 at 25 |
| 17 | 100 at 75 |
| 16 | 100 at 25 |
| 14 | 100 at 100 |
| 13 | 100 at 25 |
| 12 | 100 at 50 |
| 11 | 0 at 100 |
| 10 | 100 at 25 |
| 8 | 100 at 5 |
| 7 | 100 at 75 |
| 6 | 0 at 100 |
| 5 | 0 at 100 |
| 4 | 0 at 100 |
| 3 | 100 at 75 |
| 2 | 100 at 100 |
| 1 | 100 at 100 |

Magainin I analogues were also prepared wherein amino acid residues 19, 16, or 8, respectively, were substituted with alanine residues, and such substitution analogues were tested for % effective growth inhibition of S. epidermidis and/or S. aureus as compared to Magainin I without any substitutions. Magainin I had a % effective growth inhibition of S.epidermidis of 100% at 25 µg/ml and of S.aureus of 80% at 100 µg/ml. The % effective growth inhibition of S. epidermidis for the substitution analogue of Magainin I wherein amino acid residue 19 was substituted with alanine was 100% at 25 µg/ml, and for the substitution analogue of Magainin I wherein amino acid residue 16 was substituted with an alanine residue was 75% at 100 µg/ml. The % effective growth inhibition of S. aureus for the substitution analogue of Magainin I wherein amino acid residue 19 was substituted with alanine was 100% at 25 µg/ml, and for the substitution analogue of Magainin I wherein amino acid residue 8 was substituted with alanine was 100% at 50 µg/ml.

### Example 4 - Magainin I analogues with Proline Substitutions (comparative)

Magainin I analogues wherein a proline residue was substituted for various amino acid residues of Magainin I were prepared and tested as hereinabove described for % effective growth inhibition of E. coli at a concentration given in µg/ml. The % effective growth inhibition for each of the proline-substituted analogues of Magainin I is listed below in Table II.

**Table II -**

| Proline-Substituted Magainin I Analogues | |
|---|---|
| Amino Acid Residue Substituted | % Effective Growth Inhibition Concentration in µg/ml |
| 22 | 0 at 100 |
| 21 | 100 at 25 |
| 20 | 100 at 100 |
| 19 | 100 at 50 |
| 18 | 100 at 75 |
| 17 | 0 at 100 |
| 16 | 0 at 100 |
| 15 | 50 at 100 |
| 14 | 0 at 100 |
| 13 | 100 at 100 |
| 12 | 0 at 100 |
| 11 | 0 at 100 |
| 10 | 0 at 100 |
| 9 | 100 at 100 |
| 8 | 100 at 75 |
| 7 | 0 at 100 |
| 6 | 0 at 100 |
| 4 | 0 at 100 |
| 3 | 100 at 75 |
| 2 | 0 at 100 |
| 1 | 100 at 100 |

### Example 5 - Lysine-Substituted Magainin I Analogues

Magainin I analogues wherein a lysine residue was substituted for various amino acid residues of Magainin I were prepared and tested as hereinabove described for % effective growth inhibition of E.coli at a concentration given in µg/ml. The % effective growth inhibition of each lysine-substituted analogue of Magainin I is given below in Table III.

**Table III -**

| Lysine-Substituted Analogues of Magainin I | |
|---|---|
| Amino Acid Residue Substituted | % Effective Growth Inhibition Concentration in µg/ml |
| 23 | 100 at 5 |
| 21 | 100 at 25 |
| 20 | 100 at 25 |
| 19 | 100 at 2.5 |
| 18 | 100 at 25 |
| 17* | 100 at 100 |
| 16* | 0 at 100 |
| 15* | 100 at 50 |
| 13* | 100 at 50 |
| 12* | 100 at 100 |
| 10 | 100 at 25 |
| 9* | 100 at 100 |
| 8 | 100 at 5 |
| 7 | 100 at 5 |
| 6* | 0 at 100 |
| 5* | 0 at 100 |
| 3 | 100 at 25 |
| 2* | 100 at 100 |
| 1* | 100 at 50 |

| | |
|---|---|
| * comparative example | |

Magainin I analogues wherein amino acid residues 23, 19, 8, or 7, respectively, were substituted with a lysine residue were tested for % effective growth inhibition of S. epidermidis and/or S. aureus. The substitution analogue of Magainin I wherein amino acid residue 23 was substituted with a lysine residue had a % effective growth inhibition of S. aureus of 30% at 100 µg/ml. The substitution analogue of Magainin I wherein amino acid residue 19 was substituted with a lysine residue had a % effective growth inhibition of S. epidermidis of 100% at 25 µg/ml, and of S. aureus of 50% at 100 µg/ml. The substitution analogue of Magainin I wherein amino acid residue 8 was substituted with a lysine residue had a % effective growth inhibition of S. aureus of 70% at 100 µg/ml. The substitution analogue of Magainin I wherein amino acid residue 7 was substituted with a lysine residue had a % effective growth inhibition of S. epidermidis of 100% at 25 µg/ml, and of S. aureus of 100% at 50 µg/ml.

### Example 6 - Glutamic Acid-Substituted Magainin I Analogues (comparative)

Magainin I analogues wherein a glutamic acid residue was substituted for various amino acid residues of Magainin I were prepared and tested for % effective growth inhibition of E.coli according to the methods hereinabove described. The % effective growth inhibition for each glutamic acid-substituted Magainin I analogue is described below in Table IV.

**Table IV**

| Glutamic Acid-Substituted Analogues of Magainin I | |
|---|---|
| Amino Aicd Residue Substituted | % Effective Growth Inhibition Concentration in µg/ml |
| 22 | 0 at 100 |
| 21 | 100 at 75 |
| 20 | 0 at 100 |
| 18 | 100 at 75 |
| 17 | 0 at 100 |
| 16 | 0 at 100 |
| 15 | 0 at 100 |
| 14 | 0 at 100 |
| 13 | 0 at 100 |
| 12 | 0 at 100 |
| 11 | 0 at 100 |
| 10 | 100 at 75 |
| 9 | 0 at 100 |
| 8 | 100 at 75 |
| 7 | 32 at 100 |
| 3 | 0 at 100 |

### Example 7 - Magainin I peptides containing D-amino acid residues (comparative)

Magainin I peptides were synthesized wherein in each peptide one of amino acid residues 23, 19, 17, 16, 14, 4, or 2, respectively, is substituted with a D-amino acid residue. Each of these Magainin I peptide analogues was then tested as hereinabove described for % effective growth inhibition of E.coli in µg/ml. The % effective growth inhibition for each of the D-amino acid residue-subsituted analogues of Magainin I is listed below in Table V.

**Table V -**

| D-amino acid residue-substituted Magainin I Analogues | |
|---|---|
| Amino Acid Residue Substituted | % Effective Growth Inhibition Concentration in µg/ml |
| 23 | 100 at 10 |
| 19 | 100 at 25 |
| 17 | 100 at 50 |
| 16 | 100 at 50 |
| 14 | 100 at 50 |
| 4 | 100 at 25 |
| 2 | 100 at 25 |

### Example 8

In this example, a substitution analogue of Magainin I was prepared wherein amino acid residue 19, (E, or glutamic acid) was deleted, and amino acid residues 5, 8, 9, and 16 are each substituted with a lysine residue, amino acid residue 21 is substituted with a leucine residue, and amino acid residues 18 and 23 are each substituted with an alanine residue. The substitution analogue had the following structure:

GIGKKLHKKGKFGKFKGAILKA

The % effective growth inhibition of E.coli for this peptide was tested and found to be 100% at 5 µg/ml.

### Example 9

In this example, a substitution analogue of Magainin I was prepared wherein amino acid 21 was deleted and amino acid residues 5, 10, 18, and 19 were each substituted with a lysine residue, amino acid residue 7 was substituted with a phenylalanine residue, and amino acid 22 was substituted with an alanine residue. The peptide had the following structure:

GIGKKLFSAKKFGKAFVKKIAS

The % effective growth inhibition of E.coli for this peptide was tested and found to be 100% at 2.5 µg/ml.

### Example 10 (comparative)

Magainin I and Magainin II peptides were prepared wherein phenylalanine residues 5 and 12 in each peptide were iodinated. The % effective growth inhibition of E.coli for each peptide, containing the iodinated phenylalanine residues, was tested. For the Magainin I peptide, the % effective growth inhibition was 100% at 10 µg/ml. For the Magainin II peptide, the % effective growth inhibition was 100% at 5 µg/ml.

### Example 11 - Tryptophan (Formyl Group Removed)-Substituted Analogues of Magainin I (comparative)

Magainin I analogues were prepared wherein a tryptophan residue was substituted for various amino acid residues of Magainin I. The tryptophan had the formyl group removed, thus resulting in the substitution of unprotected tryptophan residues into the Magainin I analogues. % effective growth inhibition of E.coli and S. epidermidis was tested for these analogues. % effective growth inhibition of E. coli and S. epidermidis in µg/ml is listed below in Table VI.

**Table VI -**

| Tryptophan (Formyl Group Removed) - Substituted Analogues of Magainin I | | |
|---|---|---|
| Amino Acid Residue Substituted | % Effective Growth Inhibition Concentration in µg/ml | |
| | E.coli | S. epidermidis |
| 1 | 100 at 50 | 80 at 75 |
| 3 | 100 at 25 | 100 at 50 |
| 4 | 90 at 100 | 0 at 100 |
| 5 | 100 at 25 | 80 at 100 |
| 6 | 100 at 50 | 50 at 75 |
| 8 | 100 at 100 | 100 at 50 |
| 9 | 100 at 25 | 40 at 100 |
| 10 | 100 at 25 | 100 at 50 |
| 11 | 0 at 100 | 0 at 100 |
| 12 | 100 at 25 | 0 at 100 |
| 13 | 100 at 25 | 100 at 50 |
| 14 | N/A | 0 at 100 |
| 15 | 96 at 10 | 90 at 100 |
| 16 | 95 at 50 | 0 at 100 |
| 17 | 100 at 25 | 100 at 100 |
| 18 | 93 at 25 | 90 at 100 |
| 19 | 100 at 10 | 100 at 75 |
| 20 | 90 at 25 | 30 at 100 |
| 21 | 100 at 50 | 0 at 100 |
| 22 | 40 at 75 | 0 at 100 |

### Example 12 - Tryptophan (containing formyl group)-Substituted Magainin I Analogues (comparative)

Magainin I analogues were prepared wherein a tryptophan residue was substituted for various amino acid residues of Magainin I, as in Example 11, except that in this example, the formyl group remained on the tryptophan residue thus resulting in the substitution of protected tryptophan residues into the Magainin I analogues. % effective growth inhibition of E.coli and S. epidermidis was tested for each of these analogues. % effective growth inhibition of E.coli and S. epidermidis in µg/ml is listed below in Table VII.

**Table VII -**

| Tryptophan (containing formyl group)-Substituted Analogues of Magainin I | | |
|---|---|---|
| Amino Acid Residue Substituted | % Effective Growth Inhibition Concentration in µg/ml | |
| | E. coli | S. epidermidis |
| 1 | 100 at 50 | 100 at 75 |
| 3 | 100 at 50 | 100 at 75 |
| 4 | 70 at 100 | 0 at 100 |
| 5 | 100 at 50 | 100 at 100 |
| 6 | 100 at 50 | 100 at 100 |
| 7 | 100 at 75 | 90 at 100 |
| 8 | 100 at 25 | 100 at 75 |
| 9 | 100 at 50 | 44 at 100 |
| 10 | 100 at 25 | 100 at 75 |
| 11 | 0 at 100 | 0 at 100 |
| 12 | 100 at 25 | 90 at 75 |
| 13 | 100 at 10 | 100 at 50 |
| 14 | 0 at 100 | 0 at 100 |
| 15 | 100 at 10 | 100 at 75 |
| 16 | 100 at 50 | 0 at 100 |
| 17 | 100 at 50 | 100 at 75 |
| 18 | 100 at 25 | 63 at 100 |
| 19 | 100 at 75 | N/A |
| 20 | 100 at 25 | 55 at 100 |
| 21 | 92 at 50 | 36 at 100 |
| 22 | 80 at 75 | 0 at 100 |

### Example 13 - Multiple-Substitution Analogues of Magainin I with Lysine and/or alanine Residues

Analogues of Magainin I were prepared wherein amino acid residues 3, 8, 9, 16, 19, and/or 23 were substituted with alanine (A) or lysine (K) residues. % effective growth inhibition of E. coli, S. epidermidis, and/or S. aureus was tested for each substitution analogue. The structure of each synthesized substitution analogue as well as the % effective growth inhibition of E. coli, S. epidermidis, and S. aureus in µg/ml are given below in Table VIII.

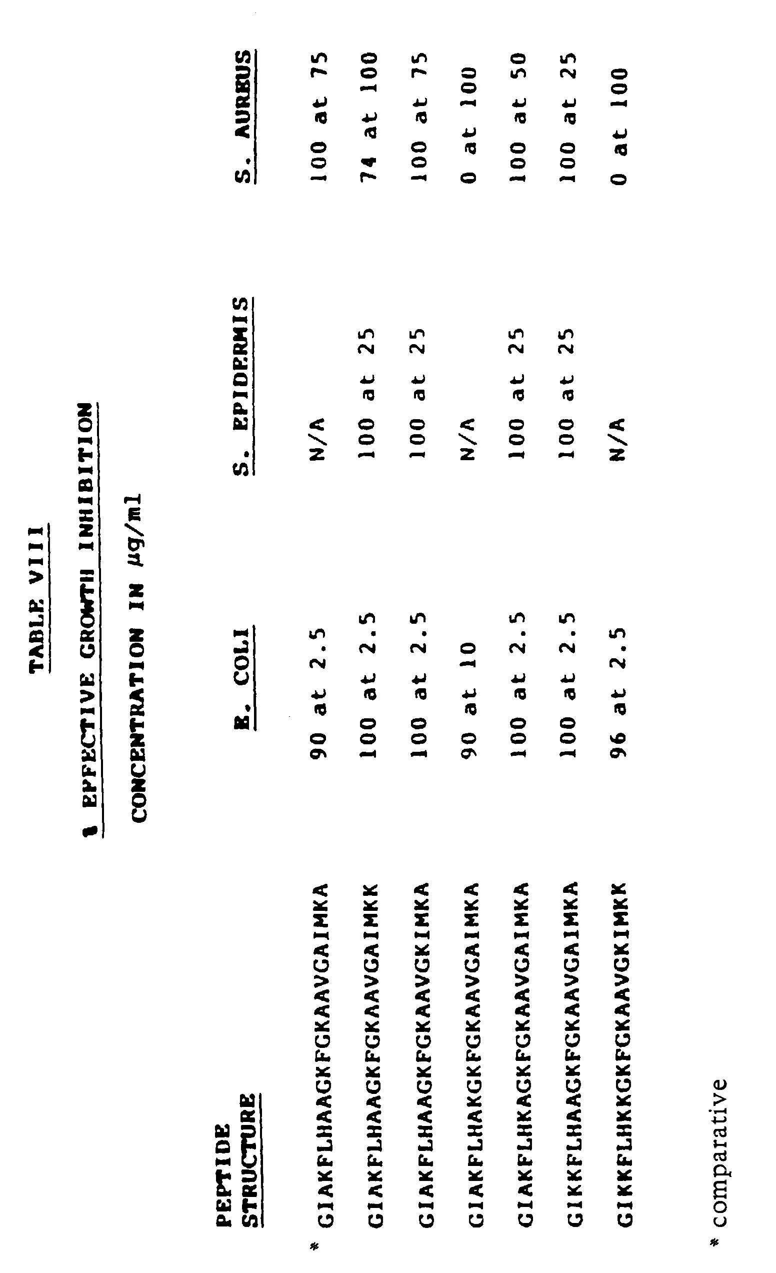

### Example 14 - Magainin I Analogues with Glutamic Acid Omissions and Alanine or Lysine Substitutions

Magainin I analogues were prepared wherein amino acid residue 19 (E, or glutamic acid) was deleted, and either alanine or lysine was substituted for one of the remaining amino acid residues in the peptide. Alanine was substituted for either residue 16, 10 or 8, or lysine was substituted for either residue 18, 8, or 7. Each of these substitution analogues was tested for % effective growth inhibition of E. coli, S. epidermidis, and/or S. aureus. The structures of each peptide and the % effective growth inhibition of E. coli, S. epidermidis, or S. aureus in µg/ml are listed below in Table IX.

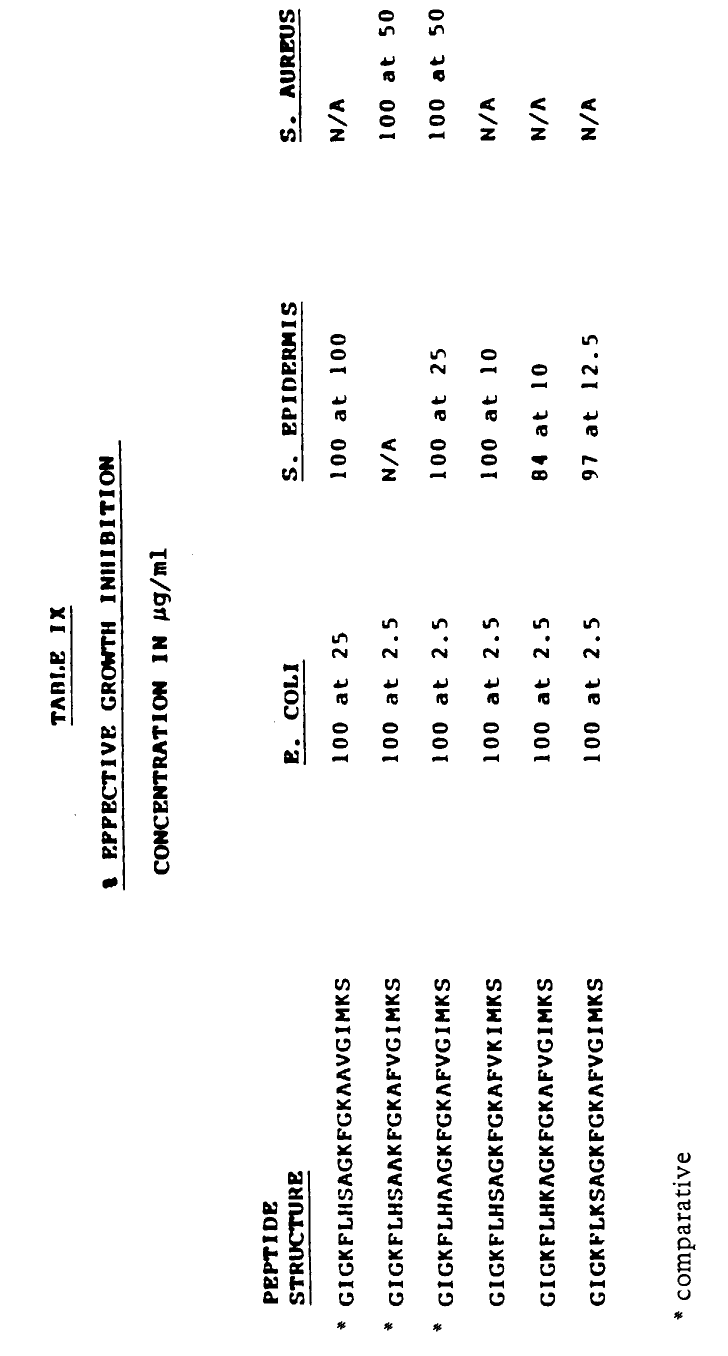

### Example 15

Magainin I substitution analogues were prepared in which, in one analogue, each of amino acid residues 8, 19, and 23 was substituted with lysine, and, in another analogue each of amino acid residues 7, 8, 19, and 23 was substituted with lysine. % effective growth inhibition of E. coli and S. epidermidis in µg/ml was then measured. For the analogue in which amino acid residues 8, 19, and 23 were substituted with lysine, the % effective growth inhibition of E. coli was 100% at 2.5 µg/ml and of S. epidermidis was 100% at 12.5 µg/ml, for the analogue in which amino acid residues 7, 8, 19, 23 were substituted with lysine, the % effective growth inhibition of E. coli was 100% at 1.25 µg/ml and the % effective growth inhibition of S. epidermidis was 100% at 5 µg/ml.

### Example 16

In this example, an analogue of Magainin I was prepared wherein amino acid residues 16 through 23 were deleted and residues 3, 7, and 8 were substituted with lysine residues. The resulting peptide had the following structure:

GIKKFLKKAGKFGKA

The peptide was then tested for % effective growth inhibition of E. coli. The % effective growth inhibition of E. coli for this peptide was found to be 100% at 5 µg/ml.

### Example 17 (comparative)

In this example, an analogue of Magainin I was prepared wherein amino acid residues 16 through 23 were deleted and amino acid residues 3, 8, and 10 were substituted with an alanine residue. The resulting peptide has the following structure:

GIAKFLHAAAKFGKA

The peptide was tested for % effective growth inhibition of E. coli. The % effective growth inhibition of E. coli for this peptide was found to be 100% at 25 µg/ml.

### Example 18 - Magainin II Analogues with Alanine Substitutions (comparative)

Magainin II and analogues wherein an alanine residue was substituetd for various amino acid residues of Magainin II were prepared as hereinabove described in Example 1 and tested for % effective growth inhibition of E-Coli, P.aeruginosa, and S. epidermidis as hereinabove described in Example 2. The % effective growth inhibition at a concentration in µg/ml for the alanine - substituted Magainin II analogues is listed below in Table X.

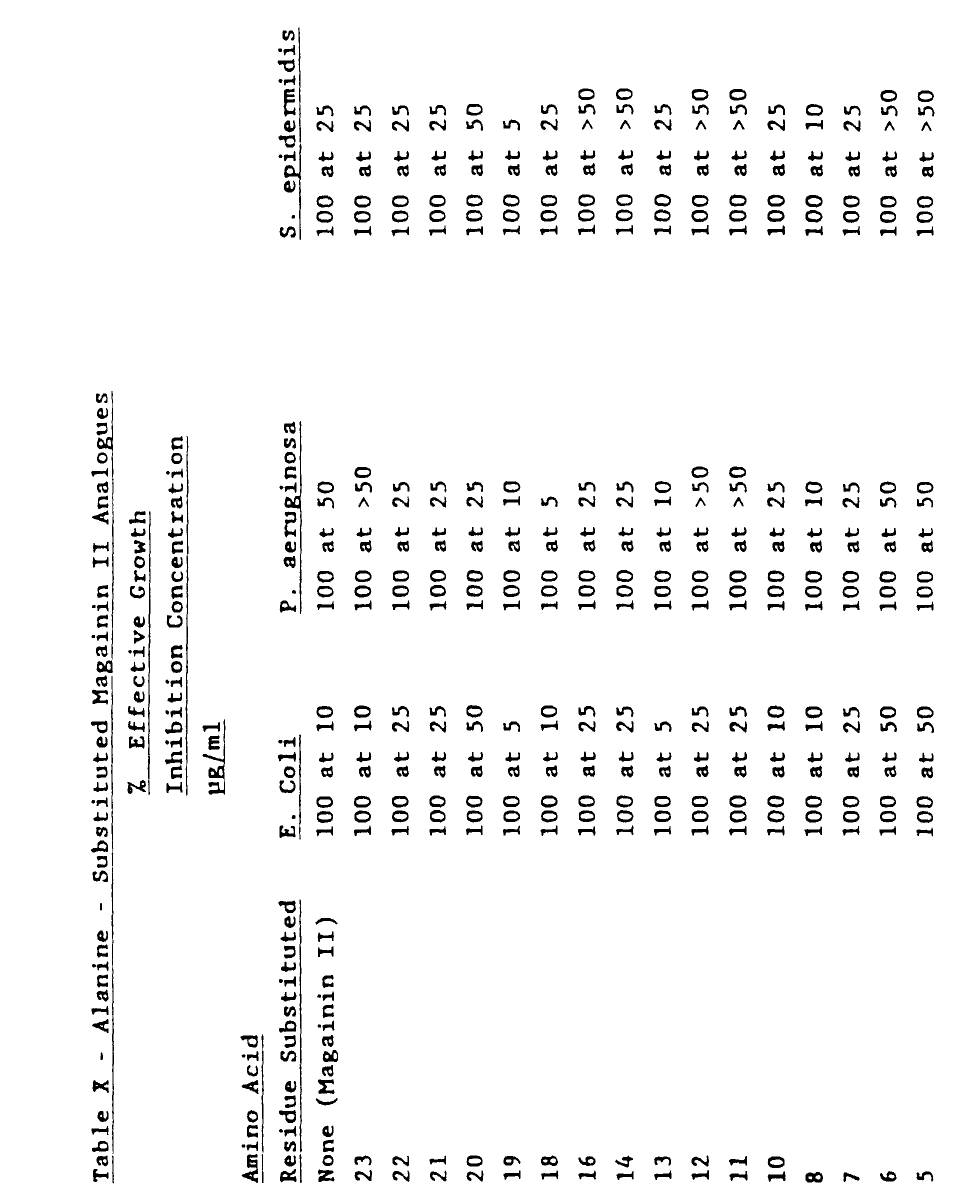

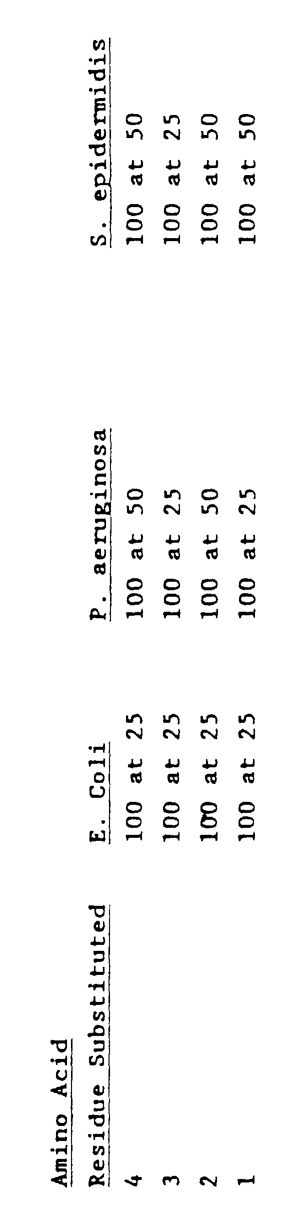

### Example 19 - Magainin II Analogues with Lysine Substiutions

Magainin II analogues wherein a lysine residue was substituted for various amino acid residues of Magainin II where prepared and tested as hereinabove described for % effective growth inhibition of E. Coli, P. aeruginosa and S. epidermidis at concentrations given in µg/ml. The % effective growth inhibition for each of the Lysine-substituted analogue of Magainin II is listed below in Table XI.

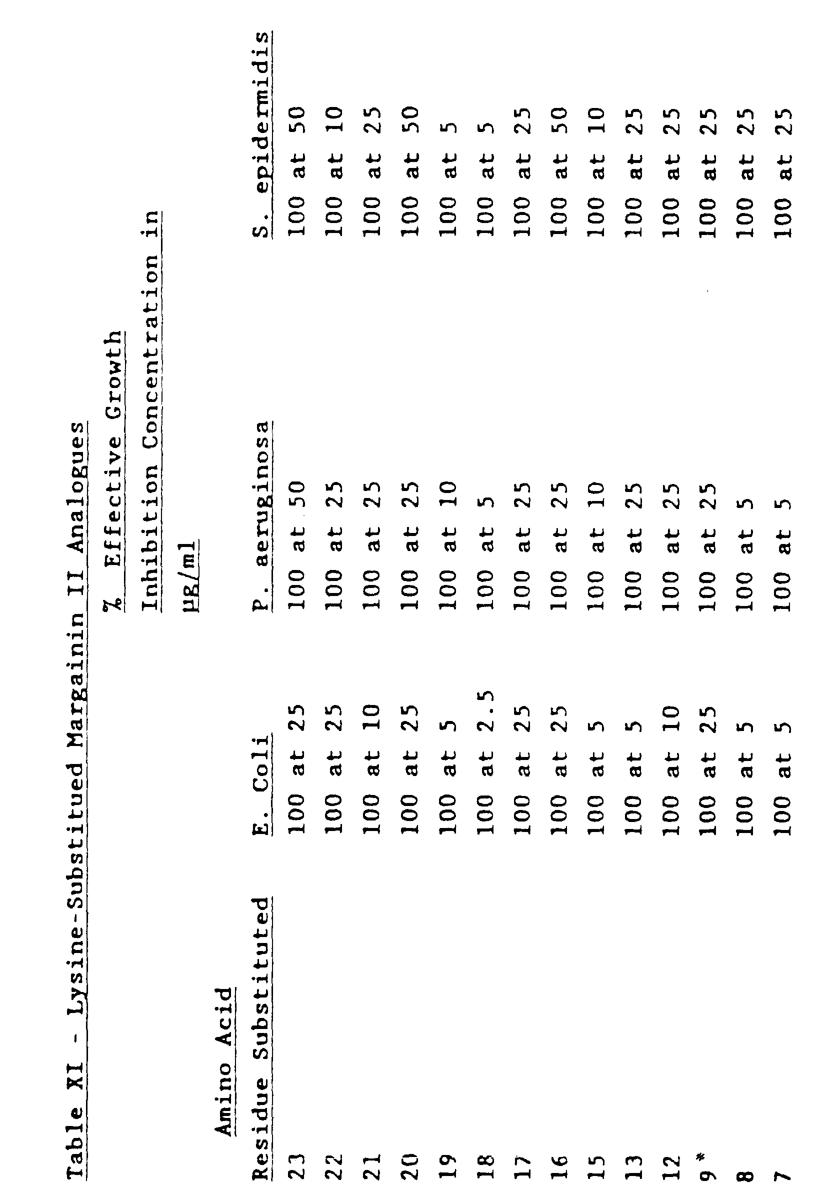

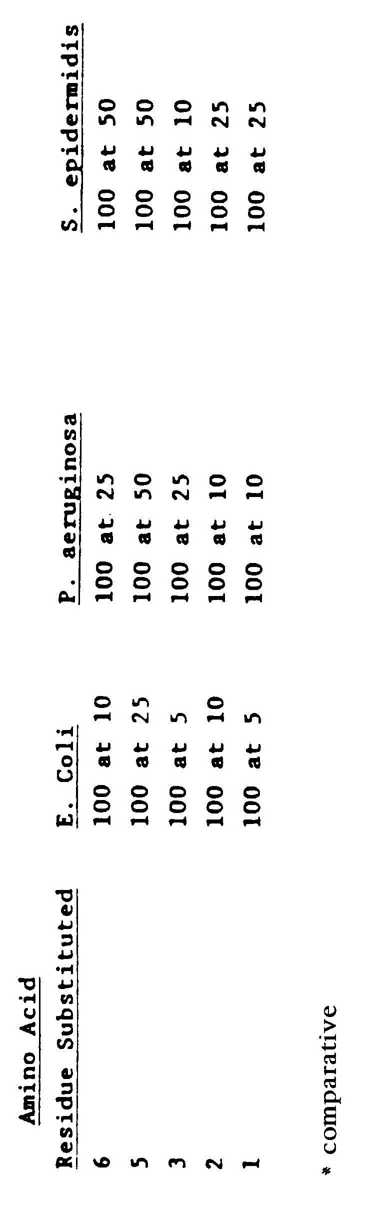

### Example 20 - Magainin II Peptides Containing D-Amino Acid Residues. (comparative)

Magainin II peptides were synthesized wherein various amino acid residues were substituted with a D-amino acid residue, whose structure corresponded to to that of the residue originally present in Magainin II. Each of these Magainin II peptide analogues was then tested as hereinabove described for % effective growth inhibition of E. Coli, P. aeruginosa, and S. epidermidis in µg/ml. The % effective growth inhibition for each of the D-Amino acid residue substituted analogues of Magainin II is listed below in Table XII.

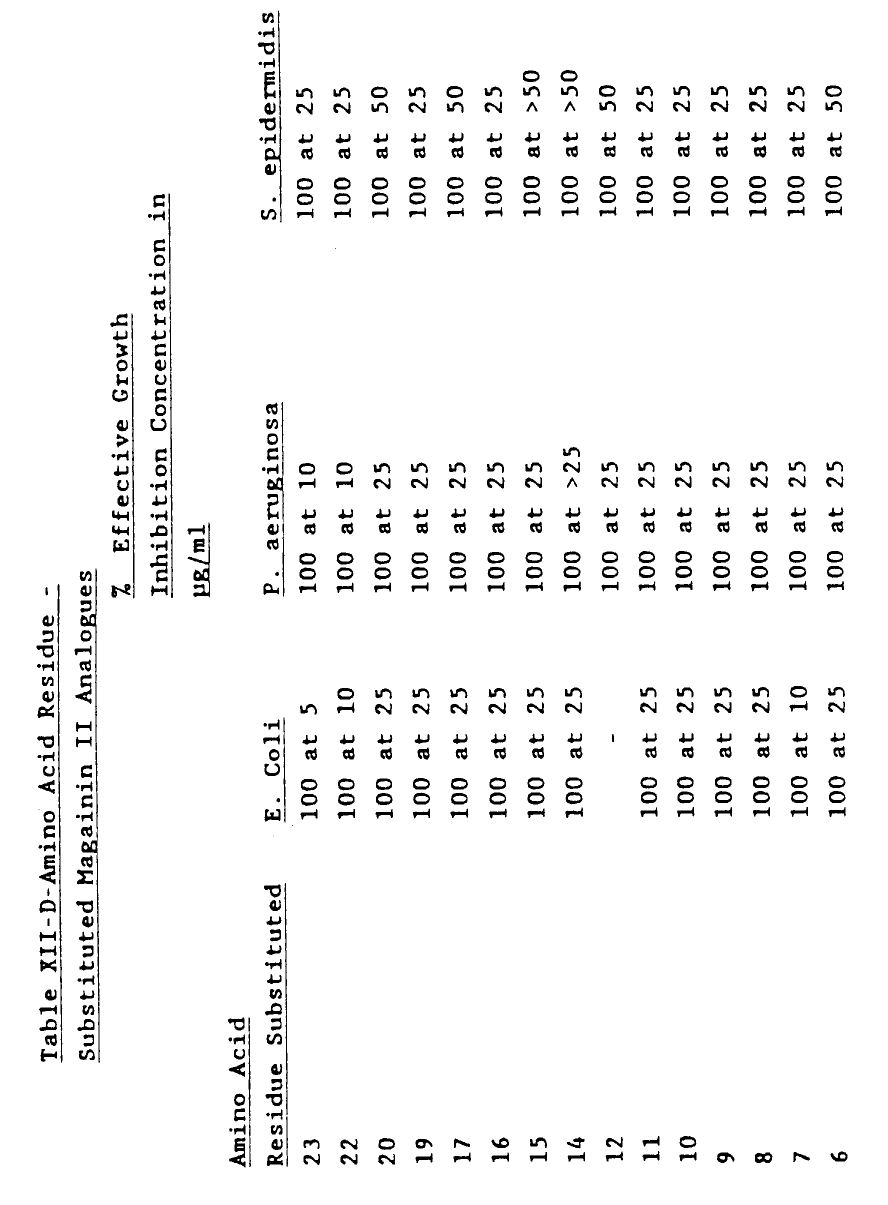

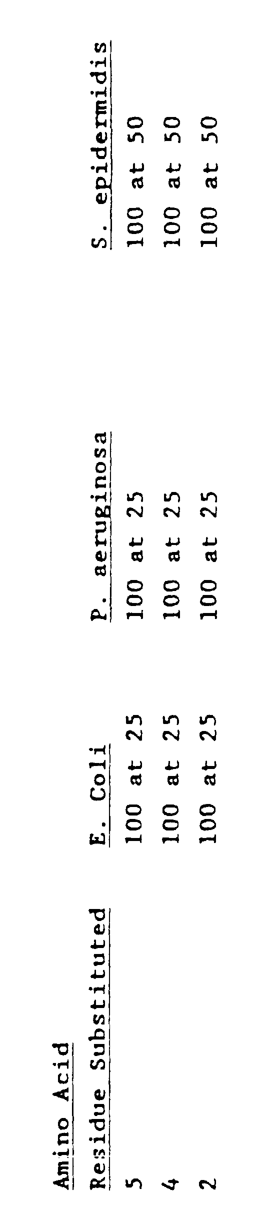

### Example 21 - Magainin II Analogues with Glutamic Acid Deletions

In this example, analogues of Magainin II were synthesized, said analogues having amino acid residue 19 (glutamic acid) deleted from the peptide structure, and wherein one or more of the other amino acid residues was substituted with an amino acid residue different from the normal amino acid residue. Analogues of the following structures were then synthesized according to the method described in Example 1.

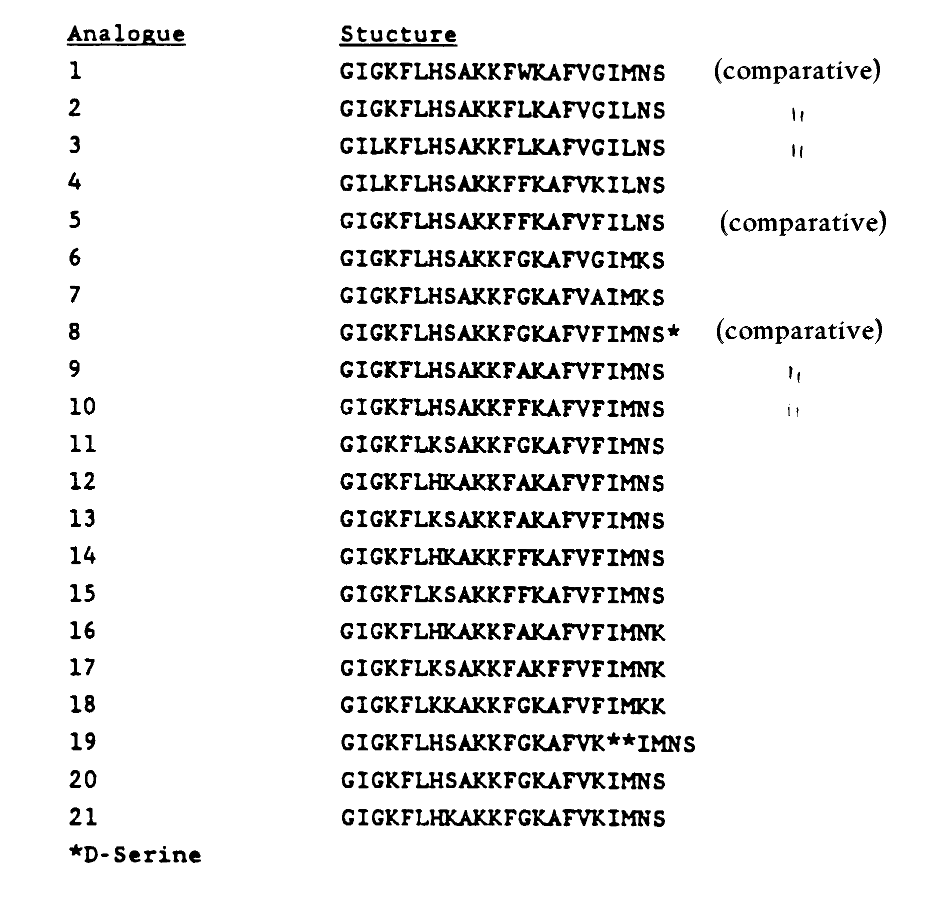

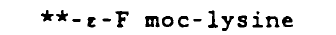

Each of the peptides was then tested as hereinabove described for % effective growth inhibition of E. Coli, P.aeruginosa, and S.epidermidis in µg/ml. The % effective growth inhibition of each of these analogues is given in Table XIII below.

**Table XIII**

| % Effective Growth Inhibition, Concentration in µg/ml | | | |
|---|---|---|---|
| Analogue | E. Coli | P. aeruginosa | S. epidermidis |
| 1 | 100 at 5 | 100 at 5 | 100 at 5 |
| 2 | 100 at 2.5 | 100 at 2.5 | 100 at 5 |
| 3 | 100 at 2.5 | 100 at 2.5 | 100 at 5 |
| 4 | 100 at 2.5 | 100 at 2.5 | 100 at 5 |
| 5 | 100 at 10 | 100 at 5 | 100 at 5 |
| 6 | 100 at 5 | 100 at 10 | 100 at 2.5 |
| 7 | 100 at 2.5 | 100 at 5 | 100 at 5 |
| 8 | 100 at 5 | 100 at 5 | 100 at 5 |
| 9 | 100 at 5 | 100 at 2.5 | 100 at 5 |
| 10 | 100 at 5 | 100 at 10 | 100 at 5 |
| 11 | 100 at 5 | 100 at 5 | 100 at 5 |
| 12 | 100 at 1.25 | 100 at 2.5 | 100 at 1.25 |
| 13 | 100 at 2.5 | 100 at 1.25 | 100 at 2.5 |
| 14 | 100 at 10 | 100 at 2.5 | 100 at 2.5 |
| 15 | 100 at 5 | 100 at 5 | 100 at 1.25 |
| 16 | 100 at 1.25 | 100 at 1.25 | 100 at 1.25 |
| 17 | 100 at 1.25 | 100 at 5 | 100 at 2.5 |
| 18 | 100 at 2.5 | 100 at 2.5 | 100 at 2.5 |
| 19 | 100 at 10 | 100 at 10 | 100 at 5 |
| 20 | 100 at 1.25 | 100 at 2.5 | 100 at 1.25 |
| 21 | 100 at 1.25 | 100 at 5 | 100 at 2.5 |

### Example 22

Amide (NH₂) - terminated Magainin II analogues wherein amino acid residue 19 (glutamic acid) was deleted, and other amino acid residues were substitued. The analogues are as follows:

Analogue 1 GIGKFLKKAKKFAKAFVKIINN-NH2

Analogue 2 GIGKFLKKAAKFAKAFVKIINN-NH2

The analogues were then tested as hereinabove described for % effective growth inhibition of E. Coli, P.aeruginosa, and S.epidermidis Analogue 1 had % effective growth inhibitions of 100% at 1.25 µg/ml for E. Coli, 100% at 2.5 µg/ml for P.aeruginosa, and 100% at 1.25 µg/ml for S.epidermidis Analogue 2 had % effective growth inhibitions of 100% at 1.25 µg/ml for P. aeruginosa and 100% at 1.25 µg/ml for S.epidermidis.

### Example 23

Amide (NH₂) -terminated analogues, which by virtue of their structures, may be analogues of Magainin I or Magainin II, were synthesized wherein amino acid residue 19 (glutamic acid) was deleted, and other amino acid residues were substituted. The analogues are as follows:

Analogue 1 GIGKFLKKAKKFGKAFVKIMKK-NH2

Analogue 2 GIGKFLKKAKKFAKAFVKIMKK-NH2

Analogue 3 GIGKFLKKAKKFAKAFVKIIKK-NH2

The analogues were then tested for % effective growth inhibition. Analogue 1 had % effective growth inhibitions of 100% at 1.25 µg/ml for E. Coli, 100% at 2.5 µg/ml for P. aeruginosa, and 100% at 1.25 µg/ml for S.epidermidis. Analogue 2 had % effective growth inhibitions of 100% at 2.5 µg/ml for E.Coli, 100 at 2.5 µg/ml for P.aeruginosa, and 100% at 1.25 µg/ml for S.epidermidis. Analogue 3 had % effective growth inhibitions of 100% at 1.25 µg/ml for E. Coli, 100% at 2.5 µg/ml for P.aeruginosa, and 100% at 1.25 µg/ml for S.epidermidis

### Example 24

Amide (NH₂) terminated analogues of Magainin I were synthesized wherein at least amino acid residues 17-23 were deleted and amino acid residues 3, 7, and 8 were substituted with lysine residues. The analogues had the following structures.

Analogue 1 - GIKKFLKKAGKFGK-NH2

Analogue 2 - GIKKFLKKAGKFGKAF-NH2

The analogues were then tested for effective growth inhibition. Analogue 1 had % effective growth inhibitions of 100% at 10 µg/ml for E. Coli, 100% at 10 µg/ml for P.aeruginosa, and 100% at 10 µg/ml for S.epidermidis. Analogue 2 had % effective growth inhibitions of 100% of 2.5 µg/ml for E. Coli, 100% at 5 µg/ml for P.aeruginosa, and 100% at 5 µg/ml for S.epidermidis.

### Example 25

Amide (NH₂)-terminated analogues of Magainin II were synthesized wherein at least amino acid residues 16-23 were deleted and substitutions were made for other residues. The analogues had the following structures.

Analogue 1 - GIKKFLKKAKKFGKA-NH2

Analogue 2 - GIKKFLKKAKKFAKA-NH2

The analogues were then tested for effective growth inhibition. Analogue 1 had % effective growth inhibitions of 100% at 10 µg/ml for P.aeruginosa, and 100 at 5.0 µg/ml for S.epidermidis. Analogue 2 had % effective grwoth inhibitions of 100% of 5 µg/ml, for E. Coli, 100% at 5 µg/ml for P.aeruginosa, and 100% at 5 µg/ml for S.epidermidis.

### Example 26

An amide (NH₂)-terminated analogue, which by virtue of its structure may be an analogue of Magainin I or Magainin II, was synthesized wherein amino acids 16-23 were deleted, amino acid residues 3,7, and 8 were substituted with lysine residues, and amino acid residue 10 and 13 were substituted with alanine residues. The analogue was of the following structure.

GIKKFLKKAAKFAKA-NH2

The analogue was then tested for % effective growth inhibition. The analogue had % effective growth inhibitions of 100% at 5 µg/ml for E. Coli, 100% at 5 µg/ml for P.aeruginosa, and 100 at 5 µg/ml for S.epidermidis.

### Example 27 (comparative)

Analogues of Magainin II were synthesized wherein amino acid residue 19 (glutamic acid) was deleted, and amino acids 1-4, or 3,5, and 6 were deleted and at least one of the remaining amino acid residues was substituted. The analogues were of the following structures: Analogue 1: FLKSAKKFGKAFVGIMNS Analogue 2: FLKSAKKFAKAFVGIMNS Analogue 3: FLHSAKKFGKAFVFIMNS Analogue 4: GIKHSAKKFAKAFKAIMNS The analogues were then tested for % effective growth inhibition of E.coli, P.aeruginosa, and S.epidermidis. The % effective growth inhibition of each of these analogues is given in Table XIV below.

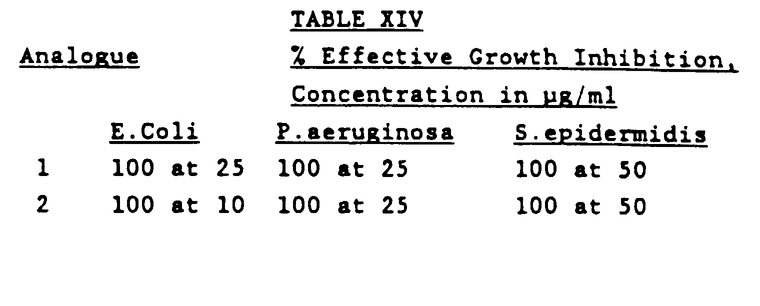

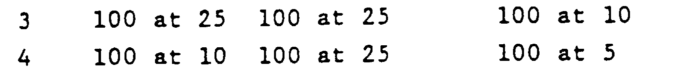

Numerous modifications and variations of the present invention are possible in light of the above teachings, and, therefore, within the scope of the accompanying claims, the invention may be practiced other than as particularly described.

## Claims

1. A compound having antimicrobial, antiviral or antitumor activity, comprising an analogue of Magainin I peptide or Magainin II peptide in an amide- or carboxy-terminated form, wherein Magainin I is represented by the following structural formula using the single letter amino acid code and the numbers below each residue refer to the position of the residue in the peptide: 
G I G K F L H S A G K F G K A F V G E I M K S
1 2 3 4 5 6 7 8 9 10 11 12 13 14 15 16 17 18 19 20 21 22 23
 and Magainin II is represented by the following structural formula using the single letter amino acid code and the numbers below each amino acid residue refer to the position of the residue in the peptide: 
G I G K F L H S A K K F G K A F V G E I M N S ,
1 2 3 4 5 6 7 8 9 10 11 12 13 14 15 16 17 18 19 20 21 22 23
 said analogue being characterised in that any of amino acid residues 15-23 of Magainin I peptide or Magainin II peptide is optionally omitted, and one to seven of the remaining amino acid residues of said Magainin peptide is substituted by another amino acid residue, at least one of amino acid residues 3, 7, 8, 10, 18-21 and 23 (in the case of Magainin I) or at least one of amino acid residues 1-3, 5-8, 12, 13 and 15-23 (in the case of Magainin II) being substituted by lysine and any further substitutions being as shown in the following table, provided that residue 18 may be substituted by Phe only if one of residues 15 to 17 or 19 to 23 is omitted:
| Residue No. | Substituent |
|---|---|
| 1 | Lys, Ala |
| 2 | Lys, Ala, D-Ile, Arg, Leu, Val, His, Met |
| 3 | Lys, Ala, Trp, Arg, His |
| 4 | D-Lys, Ala, Arg, His |
| 5 | D-Phe, Ala, Lys, Trp, Leu, Ile, Val, Met |
| 6 | D-Leu, Lys, Ala, Ile, Val, Met |
| 7 | Lys, D-His, Ala, Arg, Ile, Val, Met |
| 8 | Ala, Lys, D-Ser, Trp, Met, Ile, Arg, His, Thr, Leu, Val |
| 9 | Lys, D-Ala, Trp, Arg, His, Leu, Ile, Val |
| 10 | D-Lys, Lys, Ala, Trp, Arg, His, Leu, Ile, Val |
| 11 | D-Lys, Arg, His |
| 12 | D-Phe, Lys, Trp, Arg, His |
| 13 | Ala, Lys, Trp, Met, Arg, His, Phe, Leu, Ile, Val |
| 14 | Ala, D-Lys, Arg, His |
| 15 | Lys, Trp, D-Ala, Arg, His, Phe |
| 16 | Ala, Lys, D-Phe, Ile, Val, Met, Leu |
| 17 | Ala, Lys, D-Val, Trp, Arg, His, Met, Leu |
| 18 | Ala, Lys, Trp, Arg, His, Leu, Met, Phe |
| 19 | Ala, Lys, D-Glu, Gly, Arg, His, Leu Ile, Phe, Asn |
| 20 | D-Ile, Ala, Lys |
| 21 | Lys, Pro, Ala, His, Leu, Arg, Ile, Phe |
| 22 | Lys, Ala, D-Asn, Gln, Arg, His |
| 23 | D-Ser, Lys, Ala, Thr, Gly, Leu, Ile, Gln, Asn |

2. A compound according to claim 1 wherein amino acid residue 19 is omitted, provided that at least one of amino acid residues 3, 7, 8, 10, 13, 15, 16, 18, 21, 22 or 23 is substituted as shown in the following table:
| Residue No. | Substituent |
|---|---|
| 3 | Lys |
| 7 | Lys |
| 8 | Lys, Ala |
| 10 | Lys, Ala |
| 13 | Trp, Phe, Leu, Ala |
| 15 | Phe |
| 16 | Ala |
| 18 | Lys, Ala, Phe |
| 21 | Lys, Ile |
| 22 | Lys |
| 23 | Lys, D-Ser |

3. A compound according to claim 1, wherein at least one of amino acid residues 15-23 is omitted.

4. A compound according to any of claims 1 to 3, which is an analogue of Magainin II peptide.

5. The compound of claim 1 wherein said analogue of Magainin I peptide or Magainin II peptide is selected from: GIGKKLHKKGKFGKFKGAILKA; GIKKFLKKAGKFGKA; GIGKFLKKAKKFAKAFVKIINN-NH2; GIGKFLKKAAKFAKAFVKIINN-NH₂; GIGKFLKKAKKFGKAFVKIMKK-NH2; GIGKFLKKAKKFAKAFVKIMKK-NH2; GIGKFLKKAKKFAKAFVKIIKK-NH2; GIKKFLKKAGKFGK-NH2; GIKKFLKKAGKFGKAF-NH2; GIKKFLKKAKKFGKA-NH2; GIKKFLKKAKKFAKA-NH2; and

GIKKFLKKAAKFAKA-NH₂.

6. A process comprising:
administering to a host an effective spermicidal amount of the compound of any of claims 1 to 5.

7. The use of a compound according to any of claims 1 to 5 in the manufacture of a medicament for treating microbial infections.

8. The use of a compound according to any of claims 1 to 5 in the manufacture of a medicament for treating viral infections.

9. The use of a compound according to any of claims 1 to 5 in the manufacture of a medicament for treating tumors.

## Revendications

1. Composé ayant une activité antimicrobienne, antivirale ou antitumeur, comprenant un analogue de petitde de magainine I ou de peptide de magainine Il sous une forme terminée par un amide ou un carboxy, dans lequel la magainine I est représentée par la formule strucutrelle suivante en utilisant le code des acides aminés à lettre unique et les nombres en dessous de chaque résidu se réfèrent à la position du résidu dans le peptide : 
G I G K F L H S A G K F G K A F V G E I M K S
1 2 3 4 5 6 7 8 9 10 11 12 13 14 15 16 17 18 19 20 21 22 23
 et la magainine II est représentée par la formule structurelle suivante en utilisant le code des acides aminés à lettre unique et les nombres en dessous de chaque résidu acide aminé se réfèrent à la position du résidu dans le peptide : 
G I G K F L H S A K K F G K A F V G E I M N S
1 2 3 4 5 6 7 8 9 10 11 12 13 14 15 16 17 18 19 20 21 22 23
 ledit analogue étant caractérisé en ce que l'un des résidus acide aminé 15 à 23 du peptide de magainine I ou du peptide de magainine Il est optionnellement omis, et un à sept des résidus acide aminé restants dudit peptide de magainine est substitué par un autre résidu acide aminé, au moins un des résidus acide aminé 3, 7, 8, 10, 18 à 21 et 23 (dans le cas de la magainine I) ou au moins un des résidu acide aminé 1 à 3, 5 à 8, 12, 13 et 15 à 23 (dans le cas de la magainine II) étant substitué par la lysine et toutes les substitutions supplémentaires étant comme montrées dans le tableau suivant, à condition que le résidu 18 puisse être subsituté par Phe seulement si un des résidus 15 à 17 ou 19 à 23 est omis :
| Résidu No. | Substituant |
|---|---|
| 1 | Lys, Ala |
| 2 | Lys, Ala, D-Ile, Arg, Leu, Val, His, Met |
| 3 | Lys, Ala, Trp, Arg, His |
| 4 | D-Lys, Ala, Arg, His |
| 5 | D-Phe, Ala, Lys, Trp, Leu, Ile, Val, Met |
| 6 | D-Leu, Lys, Ala, Ile, Val, Met |
| 7 | Lys, D-His, Ala, Arg, Ile, Val, Met |
| 8 | Ala, Lys, D-Ser, Trp, Met, Ile, Arg, His, Thr, Leu, Val |
| 9 | Lys, D-Ala, Trp, Arg, His, Leu, Ile, Val |
| 10 | D-Lys, Lys, Ala, Trp, Arg, His, Leu, Ile, Val |
| 11 | D-Lys, Arg, His |
| 12 | D-Phe, Lys, Trp, Arg, His |
| 13 | Ala, Lys, Trp, Met, Arg, His, Phe, Leu, Ile, Val |
| 14 | Ala, D-Lys, Arg, His |
| 15 | Lys, Trp, D-Ala, Arg, His, Phe |
| 16 | Ala, Lys, D-Phe, Ile, Val, Met, Leu |
| 17 | Ala, Lys, D-Val, Trp, Arg, His, Met, Leu |
| 18 | Ala, Lys, Trp, Arg, His, Leu, Met, Phe |
| 19 | Ala, Lys, D-Glu, Gly, Arg, His, Leu Ile, Phe, Asn |
| 20 | D-Ile, Ala, Lys |
| 21 | Lys, Pro, Ala, His, Leu, Arg, Ile, Phe |
| 22 | Lys, Ala, D-Asn, Gln, Arg, His |
| 23 | D-Ser, Lys, Ala, Thr, Gly, Leu, Ile, Gln, Asn |

2. Composé selon la revendication 1 dans lequel le résidu acide aminé 19 est omis, à condition qu'au moins un des résidus acides aminés 3, 7, 8, 10, 13, 15, 16, 18, 21, 22 ou 23 soit substitué comme montré dans le tableau suivant :
| Résidu No. | Substituant |
|---|---|
| 3 | Lys |
| 7 | Lys |
| 8 | Lys, Ala |
| 10 | Lys, Ala |
| 13 | Trp, Phe, Leu, Ala |
| 15 | Phe |
| 16 | Ala |
| 18 | Lys, Ala, Phe |
| 21 | Lys, Ile |
| 22 | Lys |
| 23 | Lys, D-Ser |

3. Composé selon la revendication 1, dans lequel au moins un des résidus acide aminé 15 à 23 est omis.

4. Composé selon l'une quelconque des revendications 1 à 3, qui est un analogue du peptide de magainine II.

5. Composé selon la revendication 1 dans lequel ledit analogue du peptide de magainine I ou du peptide de magainine II est choisi parmi : GIGKKLHKKGKFGKFKGAILKA ; GIKKFLKKAGKFGKA ; GIGKFLKKAKKFAKAFVKIIN-NH2 ; GIGKFLKKAAKFAKAFVKIIN-NH2 ; GIGKFLKKAKKFGKAFVKIMKK-NH2 ; GIGKFLKKAKKFAKAFVKIMKK-NH2 ; GIGKFLKKAKKFAKAFVKIIKK-NH2 ; GIKKFLKKAGKFGK-NH2 ; GIKKFLKKAGKFGKAF-NH2 ; GIKKFLKKAKKFGKA-NH2 ; GIKKFLKKAKKFAKA-NH₂ ; et

GIKKFLKKAAKFAKA-NH₂.

6. Procédé comprenant :
l'administration à un hôte d'une quantité spermicide efficace du composé de l'une des revendications 1 à 5.

7. Utilisation d'un composé selon l'une quelconque des revendications 1 à 5 dans la fabrication d'un médicament pour traiter des infections microbiennes.

8. Utilisation d'un composé selon l'une quelconque des revendications 1 à 5 dans la fabrication d'un médicament pour traiter des infections virales.

9. Utilisation d'un composé selon l'une quelconque des revendications 1 à 5 pour la fabrication d'un médicament pour traiter des tumeurs.

## Patentansprüche

1. Verbindung mit antimikrobieller, antiviraler oder Antitumoraktivität, umfassend ein Analog vom Magainin-I-Peptid oder Magainin-II-Peptid in einer Form mit einem Amid- oder Carboxyterminus, wobei Magainin-I unter Verwendung des Einbuchstabenaminosäurecodes durch die folgende Strukturformel dargestellt wird und die Zahlen unter jedem Rest die Position des Restes in dem Peptid angeben: 
G I G K F L H S A G K F G K A F V G E I M K S
1 2 3 4 5 6 7 8 9 10 11 12 13 14 15 16 17 18 19 20 21 22 23,
 und Magainin-II unter Verwendung des Einbuchstabenaminosäurecodes durch die folgende Strukturformel dargestellt wird und die Zahlen unter jedem Aminosäurerest die Position des Restes in dem Peptid angeben: 
G I G K F L H S A K K F G K A F V G E I M N S
1 2 3 4 5 6 7 8 9 10 11 12 13 14 15 16 17 18 19 20 21 22 23,
 wobei das Analog dadurch gekennzeichnet ist, daß jeder der Aminosäurereste 15 bis 23 des Magainin-I-Peptides oder Magainin-II-Peptides gegebenenfalls ausgelassen wird und einer bis sieben der zurückbleibenden Aminosäurereste des Magaininpeptides durch einen anderen Aminosäurerest ersetzt wird, mindestens einer der Aminosäurereste 3, 7, 8, 10, 18-21 und 23 (im Fall von Magainin-I) oder mindestens einer der Aminosäurereste 1-3, 5-8, 12, 13 und 15-23 (im Fall von Magainin-II) durch Lysin ersetzt wird und alle weiteren Substitutionen so sind, wie in der folgenden Tabelle gezeigt, vorausgesetzt, daß Rest 18 nur durch Phe ersetzt werden kann, wenn einer der Reste 15-17 oder 19-23 ausgelassen wird:

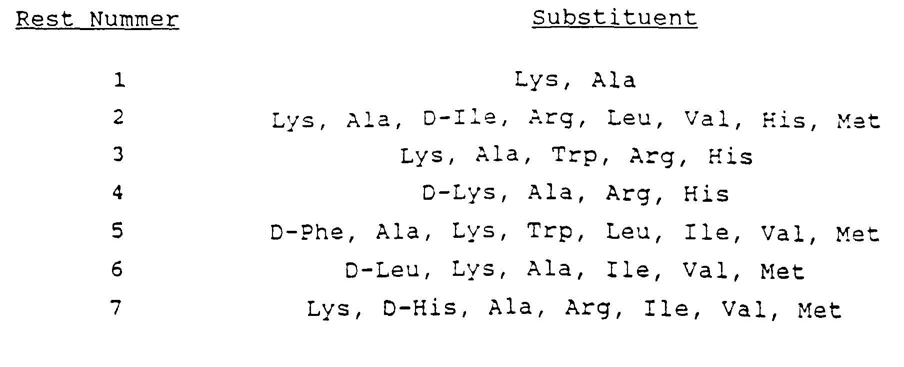

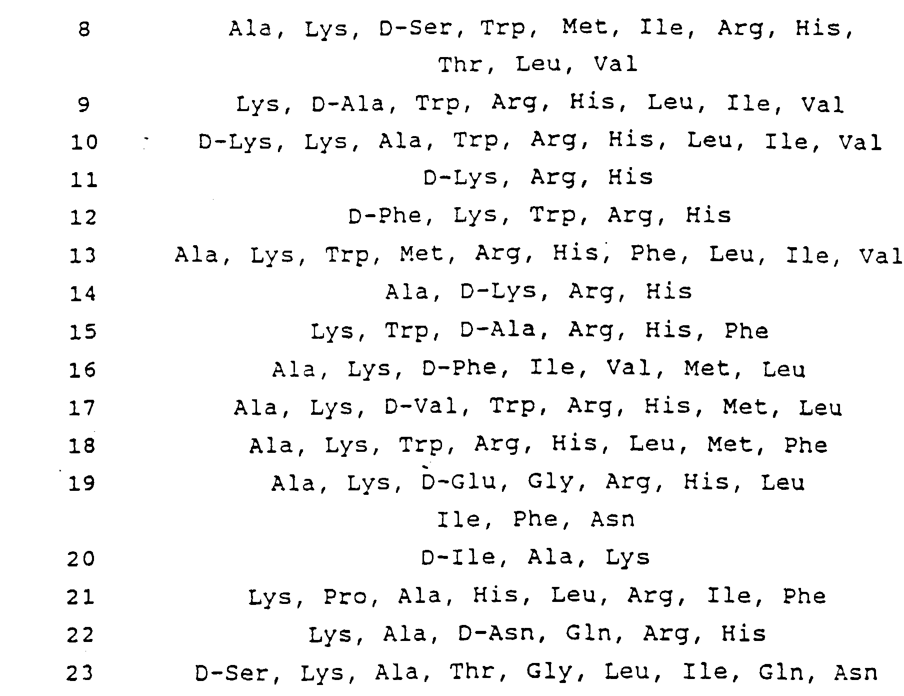

2. Verbindung nach Anspruch 1, wobei Aminosäurerest 19 ausgelassen ist, vorausgesetzt, daß mindestens einer der Aminosäurereste 3, 7, 8, 10, 13, 15, 16, 18, 21, 22 oder 23 wie in der folgenden Tabelle gezeigt ersetzt ist:
| Rest Nummer | Substituent |
|---|---|
| 3 | Lys |
| 7 | Lys |
| 8 | Lys, Ala |
| 10 | Lys, Ala |
| 13 | Trp, Phe, Leu, Ala |
| 15 | Phe |
| 16 | Ala |
| 18 | Lys, Ala, Phe |
| 21 | Lys, Ile |
| 22 | Lys |
| 23 | Lys, D-Ser |

3. Verbindung nach Anspruch 1, wobei mindestens einer der Aminosäurereste 15-23 ausgelassen ist.

4. Verbindung nach einem der Ansprüche 1 bis 3, die ein Analog des Magainin-II-Peptides ist.

5. Verbindung nach Anspruch 1, wobei das Analog des Magainin-I-Peptides oder Magainin-II-Peptides ausgewählt ist aus: GIGKKLHKKGKFGKFKGAILKA; GIKKFLKKAGKFGKA; GIGKFLKKAKKFAKAFVKIINN-NH2; GIGKFLKKAAKFAKAFVKIINN-NH₂; GIGKFLKKAKKFGKAFVKIMKK-NH2; GIGKFLKKAKKFAKAFVKIMKK-NH₂; GIGKFLKKAKKFAKAFVKIIKK-NH₂; GIKKFLKKAGKFGK-NH2; GIKKFLKKAGKFGKAF-NH2; GIKKFLKKAKKFGKA-NH2; GIKKFLKKAKKFAKA-NH₂ und

GIKKFLKKAAKFAKA-NH₂.

6. Verfahren, umfassend das Verabreichen einer wirksamen spermiziden Menge der Verbindung nach einem der Ansprüche 1 bis 5 an einen Wirt.

7. Die Verwendung einer Verbindung nach einem der Ansprüche 1 bis 3 zum Herstellen eines Medikamentes zum Behandeln mikrobieller Infektionen.

8. Verwendung einer Verbindung nach einem der Ansprüche 1 bis 5 zum Herstellen eines Medikamentes zum Behandeln von viralen Infektionen.

9. Verwendung einer Verbindung nach einem der Ansprüche 1 bis 5 zum Herstellen eines Medikamentes zum Behandeln von Tumoren.
